**Europäisches Patentamt**

**European Patent Office**

**Office européen des brevets**

(11) Numéro de publication : **0 536 290 B1**

# FASCICULE DE BREVET EUROPEEN

(45) Date de publication du fascicule du brevet :
**20.07.94 Bulletin 94/29**

(51) Int. Cl.$^5$ : **A23J 1/20**

(21) Numéro de dépôt : **91912951.0**

(22) Date de dépôt : **25.06.91**

(86) Numéro de dépôt international :
**PCT/FR91/00506**

(87) Numéro de publication internationale :
**WO 92/00017 09.01.92 Gazette 92/02**

(54) **PROCEDE ET DISPOSITIF POUR L'OBTENTION DE CASEINE BETA.**

(30) Priorité : **25.06.90 FR 9007951**

(43) Date de publication de la demande :
**14.04.93 Bulletin 93/15**

(45) Mention de la délivrance du brevet :
**20.07.94 Bulletin 94/29**

(84) Etats contractants désignés :
**AT BE CH DE DK ES FR GB GR IT LI LU NL SE**

(56) Documents cités :
**FR-A- 2 579 421
FR-A- 2 592 769
US-A- 2 702 800
Milchwissenschaft, Vol. 44, No. 8, 1989.
T.M.I.E. Christensen et al.: "Quantitative fractionation of casein by precipitation or ion exchange chromatography", pages 480-484
Patent Abstracts of Japan, Vol. 8, no. 199
(C-242)(1636), 12 September 1984, &
JP-A-5991849 (Yukijirushi Niyuugiyou K.K.) 26 May 1984**

(73) Titulaire : **EURIAL
Parc Club du Perray,
3, rue de la Rainiére,
B.P. 538
F-44077 Nantes Cédex 03 (FR)**

(72) Inventeur : **LE MAGNEN, Christine
2, rue Saint-Guillaume
F-35000 Rennes (FR)**
Inventeur : **MAUGAS, Jean-Jacques
6, rue Beaumanoir
F-35000 Rennes (FR)**

(74) Mandataire : **Peaucelle, Chantal et al
Cabinet Armengaud Aine
3, avenue Bugeaud
F-75116 Paris (FR)**

## Description

La présente invention est relative à l'obtention de la caséine bêta à partir de caséine présure. Elle vise plus particulièrement un procédé ainsi qu'un dispositif en vue de l'obtention d'une solution de caséine bêta à partir d'une suspension ou d'une solution de caséine présure.

On sait que la caséine présure est obtenue à partir de la précipitation des micelles de caséines d'un lait de mammifère après hydrolyse de la caséine par la présure ou par tout autre enzyme ou mélange d'enzymes d'origine animale, végétale, bactérienne ou fongique susceptible d'hydrolyser la liaison Phé-Met 105-106 de la caséine kappa. Après séparation du lactosérum et lavage du caillé, on obtient un produit insoluble contenant toutes les caséines du lait ainsi mis en oeuvre à l'exception de la fraction hydrophile stabilisatrice de la micelle et qui est libérée par l'enzyme à savoir le caséino macropeptide. L'autre produit de la réaction enzymatique est désigné par caséine para kappa.

Parmi les caséines présentes dans ce produit insoluble issu de la coagulation des laits de mammifères, on sait que la caséine bêta présente de nombreuses propriétés fonctionnelles, technologiques et physiologiques liées à ses caractéristiques physico-chimiques. Parmi ces propriétés on peut citer notamment :
- des propriétés moussantes et émulsifiantes qui lui sont conférées par sa forte hydrophobicité. Cette propriété intervient également dans la stabilisation de la structure micellaire en association avec le phosphate colloïdal et joue un rôle dans la texture des caillés de fromagerie ;
- des propriétés nutritionnelles résultant de sa composition en acides aminés apportant une importante richesse en lysine et tryptophane ;
- des propriétés lui permettant d'être utilisée dans l'industrie pharmaceutique ; en effet, l'hydrolyse de la caséine bêta conduit à :
    . l'obtention de phosphopeptides qui interviennent au niveau de l'absorption intestinale d'éléments minéraux et ;
    . la formation d'un hexapeptide appelé bêta casomorphine qui aurait un rôle comparable aux dérivés opiacés ayant une influence sur le sommeil, la sécrétion d'insuline et sur la régulation de l'appétit.

On sait que la caséine bêta présente la particularité de se solubiliser lorsque la température diminue et que la quantité de caséine bêta se dissociant de la micelle à froid augmente au cours du temps bien que 75 % de cette quantité soient libérés dans les quinze premières minutes (CREAMER & al, NZ J. Dairy Science Technol. 12, 58-66, 1977)

Par ailleurs, la quantité de caséine bêta susceptible de se solubiliser à froid dépend de la concentration protéique mise en oeuvre ainsi que de la concentration de phosphate colloïdal présent (PIERRE et BRULE, Journal of Dairy Research 48, 417-428, 1981).

Compte tenu des nombreuses propriétés de la caséine bêta, on comprend qu'il est important de mettre au point un procédé économiquement industrialisable, permettant la séparation de la caséine bêta des autres caséines présentes dans le milieu. Un certain nombre de tentatives ont été réalisées par le passé afin de résoudre un tel problème.

Un exemple d'une telle tentative est constitué par le brevet FR-2 592 769 qui décrit un procédé d'obtention d'une matière enrichie en caséine bêta par séparation de la caséine bêta à partir d'un lait de mammifère ou d'un dérivé de lait de mammifère, tel qu'un caséinate, ce procédé mettant notamment en oeuvre une technique de tamisage moléculaire utilisant une membrane de micro-filtration.

Cette technique connue présente un certain nombre d'inconvénients parmi lesquels on peut citer notamment les suivants :
    - une difficulté d'obtention à la fois d'une concentration élevée de caséine bêta et d'une pureté suffisante et ;
        - des débits extrêmement faibles de micro-filtration à froid, ce qui constitue un handicap certain pour l'extrapolation de ce procédé au niveau industriel.

En conséquence, la présente invention se propose d'apporter un nouveau procédé d'obtention de caséine bêta ne présentant pas les inconvénients des solutions antérieurement connues et susceptible d'être utilisé de manière industrielle. L'invention part de la constatation qu'il est possible d'extraire la caséine bêta à partir de caséine présure et qu'une telle extraction présente d'excellents rendements tant en concentration qu'en pureté.

Selon un premier aspect de cette invention, celle-ci a pour objet un procédé d'obtention d'une solution de caséine bêta à partir de caséine présure telle qu'issue de la coagulation enzymatique de lait. L'un des caractères d'originalité du procédé de l'invention par rapport à la technique antérieure décrite dans la demande FR-2592769 réside dans la mise en oeuvre non pas d'un caséinate mais d'une caséine présure dans laquelle la caséine kappa, principal contaminant rencontré lors de l'extraction de la caséine bêta, est hydrolysée en caséine para kappa présentant une solubilité beaucoup plus faible.

Ainsi, le procédé objet de la présente invention est caractérisé en ce que la suspension ou solution de caséine présure telle qu'issue de la coagulation enzymatique d'un lait de mammifère est refroidie à une température de - 2°C à + 10°C, de préférence de +2°C à + 5°C et son pH est ajusté à une valeur de 4,00 à 5,00 et en ce que la suspension ou solution de caséine présure ainsi refroidie et acidifiée est séparée en deux phases de manière à obtenir une phase solide et une phase liquide, cette dernière contenant la caséine bêta.

Dans le procédé selon l'invention, on peut utiliser notamment comme lait de mammifère du lait de vache ou du lait de chèvre et, la caséine présure provient de la coagulation enzymatique de ce lait de mammifère par un mélange d'enzymes désigné sous le terme présure ou par un enzyme d'origine animale, végétale, bactérienne, fongique ou par un mélange de ces différents enzymes permettant d'obtenir l'hydrolyse de la caséine.

Cette caséine présure peut être utilisée en suspension dans de l'eau ou dans une solution saline et elle peut se solubiliser ainsi partiellement ou totalement. Parmi les sels pouvant être utilisés, on peut citer notamment les chlorures de sodium, de potassium ou d'ammonium, les citrates de sodium, de potassium ou d'ammonium, les oxalates de sodium, de potassium ou d'ammonium, les phosphates de sodium, de potassium ou d'ammonium, ces sels pouvant être utilisés isolément ou en divers mélanges. De préférence, la concentration de sels, ou d'un mélange des différents sels utilisés dans la solution, peut varier de 0,1 à 4 %.

De préférence et selon l'invention, la concentration de caséine présure présente dans la suspension ou solution peut varier de 1 à 10 %, de préférence de 4 à 7 %.

Lors de la première étape du procédé selon l'invention, le pH de la suspension ou solution de caséine présure refroidie est ajusté à la valeur mentionnée ci-dessus de 4,00 à 5,00 par addition d'un acide organique ou d'un acide minéral ou encore d'un mélange de ces deux types d'acides. Comme acide organique, on peut utiliser notamment de l'acide acétique, de l'acide citrique, de l'acide lactique, de l'acide oxalique soit séparément soit en divers mélanges. En tant qu'acide minéral, on peut utiliser notamment l'acide chlorhydrique, l'acide sulfurique, l'acide nitrique, l'acide phosphorique, soit séparément soit en divers mélanges. Bien entendu, la valeur du pH de la suspension ou de la solution de caséine présure est contrôlée de manière à être maintenue constante pendant toute cette première phase du procédé de l'invention.

Selon la présente invention, l'étape d'acidification et de contrôle du pH de la solution ou suspension de caséine présure peut intervenir avant l'étape d'ajustement de la température aux valeurs préconisées par le procédé et précisées ci-dessus.

Après cette étape chimique du procédé, la suspension ou solution de caséine présure ainsi refroidie et ajustée en pH se présente sous la forme d'un mélange de deux phases :
- une phase liquide qui contient la caséine bêta et ;
- une phase sédimentable qui renferme le reste des caséines non solubilisées.

Dans la seconde étape du procédé selon l'invention, on réalise une séparation physique de ces deux phases soit par décantation naturelle soit à l'aide d'un appareillage approprié, comme on le verra ci-après, lors de la description d'un exemple de réalisation du dispositif pour la mise en oeuvre du procédé selon l'invention.

Après cette séparation, la phase liquide enrichie en caséine bêta est récupérée par tout moyen approprié comme on le verra ci-après.

Le procédé défini ci-dessus permet de disposer de caséine bêta ayant un degré de pureté supérieur à 90 % environ par rapport à la matière protéique totale tel que mesuré en HPLC et électrophorèse . Ce produit est avantageusement exempt d'additifs chimiques tels qu'utilisés dans les techniques de séparation connues à ce jour comme les agents dénaturants, les agents précipitants ou encore l'urée. Il s'agit d'un produit nouveau qui, en tant que tel, entre dans le cadre de l'invention.

Ce produit est plus spécialement caractérisé en ce qu'il ne contient pratiquement pas de caséine kappa.

La caséine bêta telle qu'obtenue par le procédé de l'invention est encore caractérisée en ce qu'elle présente un pH proche de la neutralité.

Les qualités de ce produit sont avantageusement mises à profit dans les industries alimentaires et diététiques, fromagères et laitières comme aliment ou complément alimentaire. En diététique, elle constitue une matière première de grand intérêt pour l'obtention de peptides.

La caséine bêta de l'invention revêt également un grand intérêt dans l'industrie pharmaceutique ou cosmétique.

L'invention vise également le co-produit de la caséine bêta isolée selon l'invention, représenté par la phase solide formée lors du refroidissement et de l'acidification de la suspension ou solution de caséine présure. Ce co-produit, pratiquement totalement exempt de caséine bêta, présente des propriétés fonctionnelles, physiologiques et nutritionnelles originales.

Il trouve donc également des applications dans les industries mentionnées plus haut. Ce co-produit peut servir également de matière première pour la purification des autres contaminants des micelles, cette purification se trouvant facilitée par le fait que ce co-produit ne renferme pratiquement pas de caséine bêta.

Selon un deuxième aspect, cette invention a pour objet un dispositif pour la mise en oeuvre du procédé, tel que défini ci-dessus, qui est caractérisé en ce qu'il comprend :

- un réacteur dans lequel on introduit la solution ou la suspension de caséine présure provenant de la coagulation enzymatique du lait de mammifère ;
- des moyens de refroidissement permettant de maintenir la température de la suspension dans le réacteur à une valeur de - 2° à + 10°C, de préférence de + 2°C à + 5°C ;
- un pH-stat permettant de réguler la quantité d'acide ajoutée dans la solution ou suspension de caséine présure contenue dans le réacteur, sous le contrôle d'un pH-mètre et ;
- des moyens assurant la séparation de la phase liquide et de la phase sédimentable contenues dans le mélange provenant dudit réacteur.

D'autres caractéristiques et avantages de la présente invention ressortiront de la description faite ci-après en référence aux dessins annexés qui en illustrent divers exemples de réalisation dépourvus de tout caractère limitatif. Sur les dessins :

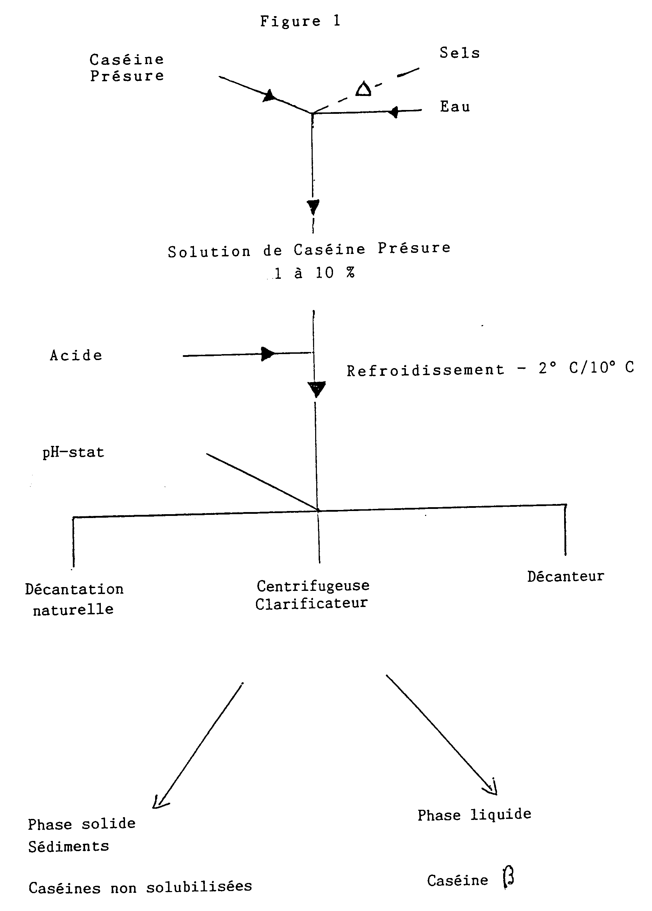

- la figure 1 est un diagramme illustrant les étapes du procédé d'obtention selon l'invention de la solution de caséine bêta ;
- la figure 2 représente schématiquement le dispositif pour la mise en oeuvre de ce procédé et ;
- les figures 3 à 5 sont des schémas illustrant les différents exemples de réalisation des moyens de séparation des deux phases provenant de la première étape du procédé de l'invention,
- la figure 6 représente le profil chromatographique d'un échantillon en HPLC.

Comme on le voit sur le dessin, la solution ou suspension de caséine présure C est introduite dans un récipient ou réacteur 10 du type à double enveloppe de manière à y maintenir la température de cette solution ou suspension à la valeur spécifiée par le procédé c'est-à-dire de - 2°C à + 10°C et de préférence de + 2°C à + 5°C. Une solution réfrigérante circule dans l'enveloppe extérieure du réacteur 10 et la solution ou suspension de caséine présure C est soumise à une faible agitation à l'aide d'un agitateur à vitesse variable 12. La température est contrôlée par exemple par un thermomètre 14.

Le pH de la solution ou suspension de caséine bêta refroidie C est maintenu à la valeur constante spécifiée par le procédé mentionné ci-dessus, à l'aide d'un pH-stat désigné dans son ensemble par la référence 16 et qui permet de réguler la quantité d'acide délivrée par une pompe doseuse 20 à partir d'un récipient d'alimentation 18 sous le contrôle d'un pH-mètre 22. On donne au pH-stat 16 une valeur de consigne et la pompe doseuse 20 permet d'ajuster le pH de la solution ou de la suspension C selon cette consigne, en délivrant dans le réacteur 10 la quantité d'acide (ou de mélange d'acides) nécessaire.

Ainsi qu'on l'a vu ci-dessus, lors de la description du procédé objet de l'invention, la suspension ou solution provenant du réacteur 10 se présente comme un mélange de deux phases : une phase liquide et une phase sédimentable et le dispositif comporte des moyens permettant de séparer ces deux phases de manière à permettre une récupération de la phase liquide contenant la caséine bêta.

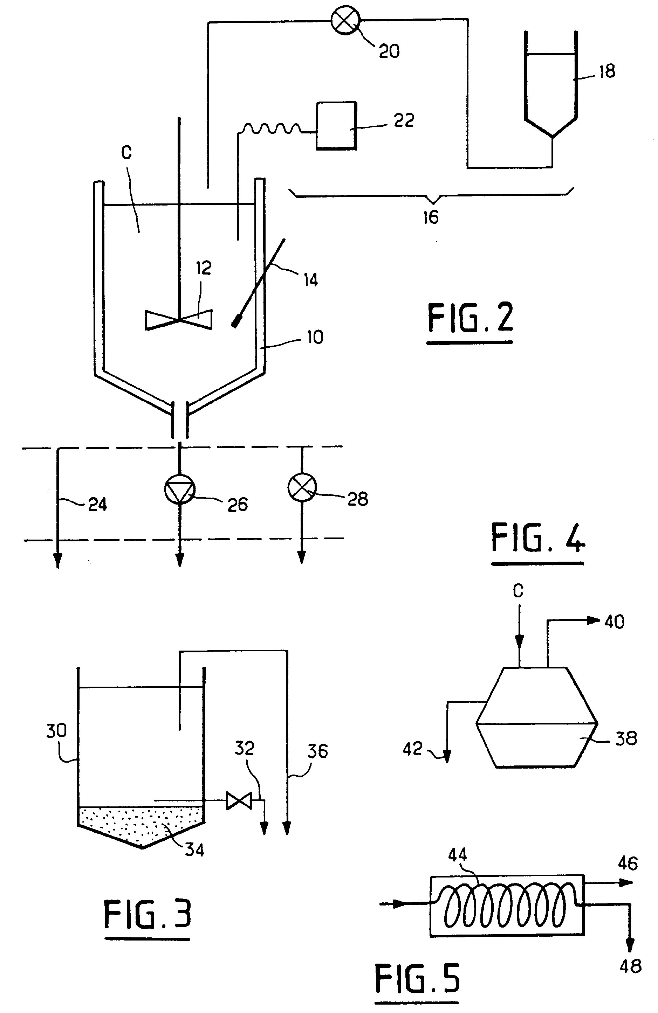

Selon la présente invention, le mélange des deux phases provenant du réacteur 10 peut être véhiculé soit directement par gravité (flèche 24 sur la figure 2) soit à l'aide d'une pompe du type volumétrique 26 ou centrifuge 28, par l'intermédiaire d'un conduit pouvant être refroidi ou non.

Les moyens qui sont utilisés pour séparer la phase liquide contenant la caséine bêta de la phase sédimentable peuvent être constitués par exemple par :

- un clarificateur du type auto-débourbeur ou non qui fonctionne sous une accélération de 500 à 8000 g, de préférence de 1500 à 3000 g ;
- un décanteur qui fonctionne avec une accélération de 500 à 8000 g et de préférence de 1500 à 3000 g ou ;
- une centrifugeuse fonctionnant selon une accélération de 500 à 8000 g;

Ces moyens peuvent être réfrigérés ou non en fonction du temps de séjour plus ou moins long des produits dans l'installation.

On peut également obtenir d'excellents résultats dans la séparation des deux phases en réalisant une décantation à la pression atmosphérique dans le récipient contenant le mélange des deux phases.

La figure 3 du dessin illustre cet exemple de réalisation . On y a représenté en 30 le récipient recevant le mélange des deux phases provenant du réacteur 10 et dans ce récipient la phase liquide qui représente le surnagent enrichi en caséine bêta est récupéré soit par un orifice 32 prévu à cet effet dans la paroi du récipient 30 immédiatement au-dessus de la phase précipité (sédiments 34 ) soit par simple siphonnage en utilisant éventuellement une pompe du type volumétrique ou centrifuge (cette possibilité est illustrée par la flèche 36 sur la figure 3).

Sur le schéma de la figure 4 on a représenté une centrifugeuse ou clarificateur 38 permettant la séparation des deux phases de la solution C provenant du réacteur 10. Dans cette variante la phase liquide contenant la caséine bêta est récupérée par exemple par siphonnage (schématisé par la flèche 40) et les sédiments sont évacués par une conduite 42.

Enfin, la schéma de la figure 5 illustre la variante mettant en oeuvre un décanteur 44 pour la séparation des deux phases. La phase liquide contenant la caséine bêta est récupérée en 46 et le sédiment évacué par la conduite 48.

Le dispositif selon l'invention décrit ci-dessus peut fonctionner soit selon un mode discontinu soit selon un mode continu.

On a donné ci-après dans un tableau des résultats d'essais réalisés par la mise en oeuvre du procédé objet de cette invention. Dans ce tableau on a mentionné cinq exemples représentatifs de l'invention concernant l'obtention d'une solution de caséine bêta à partir de caséine présure, à des concentrations différentes et en utilisant des dispositifs différents fonctionnant de façon continue ou non. Bien entendu, ces exemples ne constituent que des illustrations du procédé de l'invention.

**TABLEAU**

| Matière première | Type de séparateur | Débit m3/h | Force g | Surngt % | C g/l % | C pureté % |
|---|---|---|---|---|---|---|
| 1 Cas.présure 1,2 % | C.n.c. | | 2500 | 93 | 1,3 | > 99 |
| 2 Cas.présure 3,0 % | D c | 1 | | 90 | 3,5 | 95 |
| 3 Cas.présure | C.n.c. | | 2500 | 90 | 3,8 | 95 |
| 4 Cas.présure 5,0 % | C.n.c. | | 2500 | 86 | 5,3 | 95 |
| 5 Cas.présure | DG c | 1 | | 86 | 5,0 | > 98 |

```
n.c  = fonctionnement non continu
c    = fonctionnement continu
C    = Décanteur
DG   = Décanteur Guinard
```

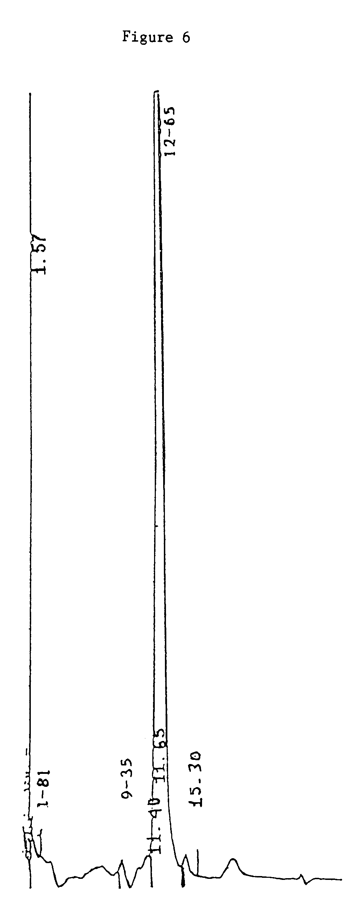

Sur la figure 6, on a représenté le profil chromatographique (analyse HPLC de phase inverse) d'un échantillon répondant à l'analyse suivante :

5

| | | |
|---|---|---|
| Solides totaux | 995 g/kg | |
| Azote | 945 g/kg | caséine bêta > 900 g/kg |
| Substance minérales | 50 g/kg | |
| Calcium | 5 g/kg | |
| Sodium | 16 g/kg | |
| Potassium | 0,1 g/kg | |
| Phosphores | 11,1 g/kg | |

Les valeurs indiquées sur la figure correspondent au temps d'élution. L'obtention d'un pic à 12,65 prouve que le produit obtenu présente une pureté supérieure à 90 %.

**Revendications**

**Revendications pour les Etats contractants suivants : AT, BE, CH, DE, DK, FR, GB, GR, IT, LI, LU, NL, SE**

1. Procédé d'obtention de caséine bêta, caractérisé en ce qu'on utilise de la caséine présure, telle qu'issue de la coagulation enzymatique de lait, dans laquelle la caséine kappa est hydrolysée en caséine para kappa.

2. Procédé d'obtention de caséine bêta à partir d'une suspension ou d'une solution de caséine présure telle qu'issue de la coagulation enzymatique d'un lait de mammifère, caractérisé en ce qu'il comprend les étapes de refroidissement de la suspension ou solution de caséine présure à une température de l'ordre de - 2°C et + 10°C, et de préférence de + 2°C à + 5°C,
   - l'ajustement du pH à une valeur de 4,00 à 5,00,
   - la séparation de la suspension ou solution de caséine présure ainsi refroidie et acidifiée en deux phases de manière à obtenir une phase solide et une phase liquide, cette dernière contenant la caséine bêta.

3. Procédé selon la revendication 1 ou 2, caractérisé en ce que la caséine présure provient de la coagulation enzymatique du lait de mammifère par un mélange d'enzymes du type présure ou par un enzyme d'origine animale, végétale, bactérienne, fongique ou un mélange de ces différents enzymes permettant de réaliser l'hydrolyse de la caséine présure.

4. Procédé selon l'une des revendications 1 à 3, caractérisé en ce que la concentration de la caséine présure présente dans la suspension ou la solution de caséine présure est de l'ordre de 1 à 10 %, de préférence de 4 à 7 %.

5. Procédé selon l'une quelconque des revendications précédentes, caractérisé en ce que la caséine présure est diluée dans de l'eau ou dans une solution saline constituée d'eau et de 0,1 à 4 % de sels choisis parmi les chlorures de sodium, de potassium ou d'ammonium, les citrates de sodium, de potassium ou d'ammonium, les oxalates de sodium, de potassium ou d'ammonium, les phosphates de sodium, de potassium ou d'ammonium, séparément ou en divers mélanges.

6. Procédé selon l'une quelconque des revendications précédentes, caractérisé en ce que le pH de la solution refroidie est ajusté par addition d'un acide organique ou d'un acide minéral ou d'un mélange de ces deux types d'acides.

7. Procédé selon la revendication 6, caractérisé en ce que l'on utilise comme acide organique l'acide acétique, l'acide citrique, l'acide lactique, l'acide oxalique, séparément ou en mélange et l'on utilise comme acide minéral l'acide chlorhydrique, l'acide sulfurique, l'acide phosphorique, l'acide nitrique, séparément ou en mélange.

8. Procédé selon l'une quelconque des revendications 2 à 7, caractérisé en ce que l'étape d'acidification de la solution ou suspension de caséine présure peut être effectuée avant l'étape de réglage de la température de cette solution ou suspension à des valeurs allant de - 2°C à + 10°C.

9. Procédé selon l'une quelconque des revendications 7 à 8, caractérisé en ce que la suspension ou solution de caséine présure, refroidie et acidifiée est séparée en deux phases : une phase liquide contenant la caséine bêta et une phase solide sédimentable contenant les autres caséines, cette séparation étant effectuée notamment par décantation à la pression atmosphérique ou par décantation clarification ou encore par centrifugation, éventuellement avec refroidissement simultané.

10. Dispositif pour la mise en oeuvre du procédé tel que défini dans l'une quelconque des revendications 1 à 9, caractérisé en ce qu'il comprend :
    - un réacteur (10) dans lequel on introduit la solution ou la suspension de caséine présure (C) provenant de la coagulation enzymatique du lait de mammifère ;
    - des moyens de refroidissement permettant de maintenir la température de la suspension dans le réacteur à une valeur de - 2°C à + 10°C, de préférence de + 2°C à + 5°C ;
    - un pH-stat (16) permettant de réguler la quantité d'acides ajoutée dans la solution ou suspension de caséine présure contenue dans le réacteur, sous le contrôle d'un pH-mètre (22) et ;
    - des moyens assurant la séparation de la phase liquide et de la phase sédimentable contenues dans le mélange provenant dudit réacteur.

11. Dispositif selon la revendication 10, caractérisé en ce que le réacteur est du type à double enveloppe, une solution réfrigérante circulant dans l'enveloppe extérieure et la suspension ou solution de caséine présure étant soumise à une faible agitation à l'aide d'un système agitateur (12) à vitesse variable.

12. Dispositif selon l'une des revendications 10 ou 11, caractérisé en ce que la valeur du pH de la solution ou suspension de caséine présure (C) contenue dans le réacteur (10) est maintenue constante par ledit pH-stat (16) auquel est donnée une valeur de consigne de pH, une pompe doseuse (20) permettant d'ajuster le pH de la solution ou suspension en fonction de la consigne en délivrant la quantité d'acide nécessaire.

13. Dispositif selon l'une quelconque des revendications 10 à 12, caractérisé en ce que la phase liquide contenant la caséine bêta est recueillie par siphonnage (36) avec éventuellement l'aide d'une pompe centrifuge ou volumétrique après sédimentation de la phase solide par décantation dans une enceinte (30) à la pression atmosphérique.

14. Dispositif selon la revendication 13, caractérisé en ce que la décantation à pression atmosphérique s'effectue dans un récipient (30) comportant un orifice situé dans la zone de l'interface entre la phase liquide et la phase sédimentée pour assurer la récupération de la phase supérieure, liquide, contenant la caséine bêta.

15. Dispositif selon l'une quelconque des revendications 10 à 12, caractérisé en ce que la séparation des deux phases est effectuée à l'aide d'une centrifugeuse (38) fonctionnant en mode continu ou non, ou encore d'un clarificateur auto-débourbeur ou non, fonctionnant en mode continu ou non, ou à l'aide d'un décanteur (44) fonctionnant en mode continu ou non.

16. Dispositif selon la revendication 15, caractérisé en ce que l'accélération des moyens de séparation des deux phases est de 500 à 8000 g.

17. Dispositif selon l'une quelconque des revendications 10 à 16, caractérisé en ce que la solution acidifiée et refroidie provenant du réacteur (10) est transmise aux moyens de séparation par gravité, par l'intermédiaire d'un conduit refroidi ou non, ou à l'aide d'une pompe volumétrique (26) ou centrifuge (28).

18. Dispositif selon l'une quelconque des revendications 10 à 17, caractérisé en qu'il fonctionne selon un mode continu ou non.

19. Caséine bêta telle qu'obtenue par le procédé selon l'une quelconque des revendications 1 à 9, caractérisée en ce qu'elle présente un degré de pureté supérieur à environ 90 % par rapport à la matière protéique totale, qu'elle est exempte d'additifs chimiques tels qu'agents dénaturants, agents précipitants ou urée, qu'elle est en outre pratiquement totalement exempte de caséine kappa et qu'elle possède un pH proche de la neutralité.

**20.** Application de la caséine bêta selon la revendication 19, comme aliment ou complément d'aliment dans l'industrie agro-alimentaire ou diététique, notamment laitière ou fromagère, ou dans l'industrie pharmaceutique ou cosmétique.

**21.** Co-produit tel que représenté par la phase solide séparée dans le procédé selon l'une quelconque des revendications 1 à 9 caractérisé en ce qu'il est exempt de caséine bêta.

**22.** Application du co-produit représenté par la phase solide séparée dans le procédé selon l'une quelconque des revendications 1 à 9 comme aliment ou complément d'aliment dans l'industrie agro-alimentaire ou diététique, notamment laitière ou fromagère, ou dans l'industrie pharmaceutique ou cosmétique.

**Revendications pour l'Etat contractant suivant : ES**

**1.** Procédé d'obtention de caséine bêta, caractérisé en ce qu'on utilise de la caséine présure, telle qu'issue de la coagulation enzymatique de lait, dans laquelle la caséine kappa est hydrolysée en caséine para kappa.

**2.** Procédé d'obtention de caséine bêta à partir d'une suspension ou d'une solution de caséine présure telle qu'issue de la coagulation enzymatique d'un lait de mammifère, caractérisé en ce qu'il comprend les étapes de refroidissement de la suspension ou solution de caséine présure a une température de l'ordre de - 2°C et + 10°C, et de préférence de + 2°C à + 5°C,
   - l'ajustement du pH à une valeur de 4,00 à 5,00,
   - la séparation de la suspension ou solution de caséine présure ainsi refroidie et acidifiée en deux phases de manière à obtenir une phase solide et une phase liquide, cette dernière contenant la caséine bêta.

**3.** Procédé selon la revendication 1 ou 2, caractérisé en ce que la caséine présure provient de la coagulation enzymatique du lait de mammifère par un mélange d'enzymes du type présure ou par un enzyme d'origine animale, végétale, bactérienne, fongique ou un mélange de ces différents enzymes permettant de réaliser l'hydrolyse de la caséine présure.

**4.** Procédé selon l'une des revendications 1 à 3, caractérisé en ce que la concentration de la caséine présure présente dans la suspension ou la solution de caséine présure est de l'ordre de 1 à 10 %, de préférence de 4 à 7 %.

**5.** Procédé selon l'une quelconque des revendications précédentes, caractérisé en ce que la caséine présure est diluée dans de l'eau ou dans une solution saline constituée d'eau et de 0,1 à 4 % de sels choisis parmi les chlorures de sodium, de potassium ou d'ammonium, les citrates de sodium, de potassium ou d'ammonium, les oxalates de sodium, de potassium ou d'ammonium, les phosphates de sodium, de potassium ou d'ammonium, séparément ou en divers mélanges.

**6.** Procédé selon l'une quelconque des revendications précédentes, caractérisé en ce que le pH de la solution refroidie est ajusté par addition d'un acide organique ou d'un acide minéral ou d'un mélange de ces deux types d'acides.

**7.** Procédé selon la revendication 6, caractérisé en ce que l'on utilise comme acide organique l'acide acétique, l'acide citrique, l'acide lactique, l'acide oxalique, séparément ou en mélange et l'on utilise comme acide minéral l'acide chlorhydrique, l'acide sulfurique, l'acide phosphorique, l'acide nitrique, séparément ou en mélange.

**8.** Procédé selon l'une quelconque des revendications 2 à 7, caractérisé en ce que l'étape d'acidification de la solution ou suspension de caséine présure peut être effectuée avant l'étape de réglage de la température de cette solution ou suspension à des valeurs allant de - 2°C à + 10°C.

**9.** Procédé selon l'une quelconque des revendications 7 à 8, caractérisé en ce que la suspension ou solution de caséine présure, refroidie et acidifiée est séparée en deux phases : une phase liquide contenant la caséine bêta et une phase solide sédimentable contenant les autres caséines, cette séparation étant effectuée notamment par décantation à la pression atmosphérique ou par décantation clarification ou encore par centrifugation, éventuellement avec refroidissement simultané.

10. Dispositif pour la mise en oeuvre du procédé tel que défini dans l'une quelconque des revendications 1 à 9, caractérisé en ce qu'il comprend :
   - un réacteur (10) dans lequel on introduit la solution ou la suspension de caséine présure (C) provenant de la coagulation enzymatique du lait de mammifère ;
   - des moyens de refroidissement permettant de maintenir la température de la suspension dans le réacteur à une valeur de - 2°C à + 10°C, de préférence de + 2°C à + 5°C ;
   - un pH-stat (16) permettant de réguler la quantité d'acides ajoutée dans la solution ou suspension de caséine présure contenue dans le réacteur, sous le contrôle d'un pH-mètre (22) et ;
   - des moyens assurant la séparation de la phase liquide et de la phase sédimentable contenues dans le mélange provenant dudit réacteur.

11. Dispositif selon la revendication 10, caractérisé en ce que le réacteur est du type à double enveloppe, une solution réfrigérante circulant dans l'enveloppe extérieure et la suspension ou solution de caséine présure étant soumise à une faible agitation à l'aide d'un système agitateur (12) à vitesse variable.

12. Dispositif selon l'une des revendications 10 ou 11, caractérisé en ce que la valeur du pH de la solution ou suspension de caséine présure (C) contenue dans le réacteur (10) est maintenue constante par ledit pH-stat (16) auquel est donnée une valeur de consigne de pH, une pompe doseuse (20) permettant d'ajuster le pH de la solution ou suspension en fonction de la consigne en délivrant la quantité d'acide nécessaire.

13. Dispositif selon l'une quelconque des revendications 10 à 12, caractérisé en ce que la phase liquide contenant la caséine bêta est recueillie par siphonnage (36) avec éventuellement l'aide d'une pompe centrifuge ou volumétrique après sédimentation de la phase solide par décantation dans une enceinte (30) à la pression atmosphérique.

14. Dispositif selon la revendication 13, caractérisé en ce que la décantation à pression atmosphérique s'effectue dans un récipient (30) comportant un orifice situé dans la zone de l'interface entre la phase liquide et la phase sédimentée pour assurer la récupération de la phase supérieure, liquide, contenant la caséine bêta.

15. Dispositif selon l'une quelconque des revendications 10 à 12, caractérisé en ce que la séparation des deux phases est effectuée à l'aide d'une centrifugeuse (38) fonctionnant en mode continu ou non, ou encore d'un clarificateur auto-débourbeur ou non, fonctionnant en mode continu ou non, ou à l'aide d'un décanteur (44) fonctionnant en mode continu ou non.

16. Dispositif selon la revendication 15, caractérisé en ce que l'accélération des moyens de séparation des deux phases est de 500 à 8000 g.

17. Dispositif selon l'une quelconque des revendications 10 à 16, caractérisé en ce que la solution acidifiée et refroidie provenant du réacteur (10) est transmise aux moyens de séparation par gravité, par l'intermédiaire d'un conduit refroidi ou non, ou à l'aide d'une pompe volumétrique (26) ou centrifuge (28).

18. Dispositif selon l'une quelconque des revendications 10 à 17, caractérisé en qu'il fonctionne selon un mode continu ou non.

19. Procédé selon l'une quelconque des revendications 1 à 9, caractérisé en ce qu'on obtient une caséine bêta présentant un degré de pureté supérieur à environ 90 % par rapport à la matière protéique totale, exempte d'additifs chimiques tels qu'agents dénaturants, agents précipitants ou urée, qui est en outre pratiquement totalement exempte de caséine kappa et possède un pH proche de la neutralité.

20. Application de la caséine bêta selon la revendication 19, comme aliment ou complément d'aliment dans l'industrie agro-alimentaire ou diététique, notamment laitière ou fromagère, ou dans l'industrie pharmaceutique ou cosmétique.

21. Procédé selon l'une quelconque des revendications 1 à 9, caractérisé en ce qu'on obtient une phase solide ou co-produit exempte de caséine bêta.

**22.** Application du co-produit représenté par la phase solide séparée dans le procédé selon l'une quelconque des revendications 1 à 9 comme aliment ou complément d'aliment dans l'industrie agro-alimentaire ou diététique, notamment laitière ou fromagère, ou dans l'industrie pharmaceutique ou cosmétique.

## Patentansprüche

### Patentansprüche für folgenden Vertragsstaaten : AT, BE, CH, DE, DK, FR, GB, GR, IT, LI, LU, NL, SE

**1.** Verfahren zur Herstellung von β-Casein, dadurch gekennzeichnet, daß man Lab-Casein verwendet, wie es bei der enzymatischen Koagulation von Milch anfällt, worin das κ-Kasein zu para-κ-Casein hydrolysiert wird.

**2.** Verfahren zur Herstellung von β-casein aus einer Suspension oder Lösung von Lab-Casein, wie es bei der enzymatischen Koagulation einer Säugetiermilch anfällt, dadurch gekennzeichnet, daß es die Stufen der Abkühlung der Suspension oder Lösung des Lab-Caseins auf eine Temperatur in der Größenordnung von -2°C bis +10°C, vorzugsweise +2°C bis +5°C,
- die Einstellung des pH-Wertes auf einen Wert von 4,00 bis 5,00,
- die Auftrennung der solcherart abgekühlten und angesäuerten Suspension oder Lösung des Lab-Caseins in zwei Phasen, um eine feste Phase und eine flüssige Phase zu erhalten, wobei die letztgenannte das β-Casein enthält,
umfaßt.

**3.** Verfahren nach Anspruch 1 oder 2, dadurch gekennzeichnet, daß das Lab-Casein aus der enzymatischen Koagulation von Säugetiermilch durch ein Enzymgemisch vom Labtyp oder durch ein Enyzm tierischer, pflanzlicher, bakterieller, pilzlicher Herkunft oder durch ein Gemisch dieser verschiedenen Enzyme stammt, die die Hydrolyse des Lab-Caseins ermöglichen.

**4.** Verfahren nach einem der Ansprüche 1 bis 3, dadurch gekennzeichnet, daß die Konzentration des Lab-Caseins, das in der Suspension oder Lösung des Lab-Caseins vorliegt, in der Größenordnung von 1 bis 10%, vorzugsweise von 4 bis 7 % liegt.

**5.** Verfahren nach einem der vorstehenden Ansprüche, dadurch gekennzeichnet, daß das Lab-Casein mit Wasser oder mit einer Salzlösung, gebildet aus Wasser und 0,1 bis 4 % Salzen, die unter Natriumchlorid, Kaliumchlorid oder Ammoniumchlorid, Natriumzitrat, Kaliumzitrat oder Ammoniumzitrat, Natriumoxalat, Kaliumoxalat oder Ammoniumoxalat, Natriumphosphat, Kaliumphosphat oder Ammoniumphosphat, einzeln oder in verschiedenen Gemischen, ausgewählt sind, verdünnt wird.

**6.** Verfahren nach einem der vorstehenden Ansprüche, dadurch gekennzeichnet, daß der pH-Wert der abgekühlten Lösung durch Zugabe einer organischen Säure oder einer Mineralsäure oder eines Gemisches dieser beiden Säuretypen eingestellt wird.

**7.** Verfahren nach Anspruch 6, dadurch gekennzeichnet, daß man als organische Säure Essigsäure, Zitronensäure, Milchsäure, Oxalsäure, einzeln oder im Gemisch, und als Mineralsäure Chlorwasserstoffsäure, Schwefelsäure, Phosphorsäure, Salpetersäure, einzeln oder im Gemisch, verwendet.

**8.** Verfahren nch einem der Ansprüche 2 bis 7, dadurch gekennzeichnet, daß die Ansäuerungsstufe der Lösung oder Suspension des Lab-Caseins vor der Temperaturregelungsstufe dieser Lösung oder Suspension auf Werte von -2°C bis +10°C ausgeführt werden kann.

**9.** Verfahren nach einem der Ansprüche 7 bis 8, dadurch gekennzeichnet, daß die abgekühlte und angesäuerte Suspension oder Lösung des Lab-Caseins in zwei Phasen aufgetrennt wird: eine flüssige Phase, die das β-casein enthält, und eine sedimentierbare feste Phase, die die anderen Caseine enthält, wobei diese Auftrennung insbesondere durch Dekantieren bei Atmosphärendruck oder durch Klärdekantation oder auch durch Zentrifugieren, gegebenenfalls unter gleichzeitiger Kühlung, ausgeführt wird.

**10.** Vorrichtung zur Durchführung des Verfahrens, wie in einem der Ansprüche 1 bis 9 definiert, dadurch gekennzeichnet, daß sie umfaßt:

- einen Reaktor (10), in welchen die Lösung oder Suspension des aus der enzymatischen Koagulation von Säugetiermilch stammenden Lab-Caseins (C) eingeführt wird;
- Kühlmittel, die eine Aufrechterhaltung der Temperatur der Suspension im Reaktor auf einem Wert von -2°C bis +10°C, vorzugsweise +2°C bis +5°C, gestatten;
- einen pH-Stat (16), der unter der Kontrolle eines pH-Meters (22) die Regelung der Säurenmenge gestat-tet, die der im Reaktor enthaltenen Lösung oder Suspension des Lab-Caseins zugesetzt wird; und
- Mittel, die eine Trennung der wäßrigen Phase und der sedimentierbaren Phase gestatten, die in dem vom Reaktor kommenden Gemisch enthalten sind.

11. Vorrichtung nach Anspruch 10, dadurch gekennzeichnet, daß der Reaktor vom Doppelmanteltyp ist, wobei im Außenmantel eine Kühllösung umläuft und die Suspension oder Lösung des Lab-Caseins mit Hilfe eines Rührsystems (12) mit variierbarer Geschwindigkeit einer schwachen Bewegung unterworfen wird.

12. Vorrichtung nach einem der Ansprüche 10 oder 11, dadurch gekennzeichnet, daß der pH-Wert der im Reaktor (10) enthaltenen Lösung oder Suspension des Lab-Caseins (C) mit dem pH-Stat (16) konstant gehalten wird, dem ein pH-Sollwert eingegeben wird, wobei eine Dosierpumpe (20) die Einstellung des pH-Werts der Lösung oder Suspension in Abhängigkeit vom Sollwert durch Abgabe der erforderlichen Säuremenge ermöglicht.

13. Vorrichtung nach einem der Ansprüche 10 bis 12, dadurch gekennzeichnet, daß die das β-Casein enthaltende flüssige Phase durch Absaugen, gegebenenfalls mit Hilfe einer Zentrifugalpumpe oder Verdrängerpumpe, nach einem Sedimentieren der festen Phase durch Dekantieren in einem Behälter (30) bei atmosphärischem Druck gewonnen wird.

14. Vorrichtung nach Anspruch 13, dadurch gekennzeichnet, daß das Dekantieren bei atmosphärischem Druck in einem Behälter (30) erfolgt, der eine im Bereich der Grenzschicht zwi-schen der flüssigen Phase und der sedimentierten Phase an-geordnete Öffnung trägt, um die Gewinnung der oberen, flüssigen, das β-casein enthaltenden Phase sicherzustellen.

15. Vorrichtung nach einem der Ansprüche 10 bis 12, dadurch gekennzeichnet, daß die Trennung der beiden Phasen mit Hilfe einer kontinuierlich oder diskontinuierlich betriebenen Zentrifuge (38), mit Hilfe einer kontinuierlich oder diskontinuierlich arbeitenden Klärvorrichtung, die selbstentschlammend ist oder auch nicht, oder mit Hilfe einer kontinuierlich oder diskontinuierlich betriebenen Dekantiervorrichtung (44) vorgenommen wird.

16. Vorrichtung nach Anspruch 15, dadurch gekennzeichnet, daß die Beschleunigung der Mittel zur Auftrennung der beiden Phasen von 500 bis 8.000 g beträgt.

17. Vorrichtung nch einem der Ansprüche 10 bis 16, dadurch gekennzeichnet, daß die vom Reaktor (10) kommende, angesäuerte und abgekühlte Lösung den Trennvorrichtungen durch Schwerkraft, durch eine zwischengeschaltete gekühlte oder ungekühlte Leitung oder mit Hilfe einer Verdrängerpumpe (26) oder Zentrifugalpumpe (28) den Trennmitteln zugeführt wird.

18. Vorrichtung nch einem der Ansprüche 10 bis 17, dadurch gekennzeichnet, daß sie kontinuierlich oder diskontinuierlich betrieben wird.

19. β-casein, erhalten nach dem Verfahren nach einem der Ansprüche 1 bis 9, dadurch gekennzeichnet, daß es einen Reinheitsgrad von über etwa 90 %, bezogen auf das Gesamtproteinmaterial, aufweist, daß es frei von chemischen Zusätzen, wie Denaturierungsmitteln, Fällungsmitteln oder Harnstoff ist, daß es darüberhinaus praktisch zur Gänze frei von κ-Casein ist und daß es einen pH-Wert nahe dem Neutralpunkt aufweist.

20. Anwendung des β-Caseins nach Anspruch 19 als Nahrungsmittel oder Nahrungsmittelergänzung in der Lebensmittelindustrie und diätetischen Industrie, insbesondere Molkerei oder Käserei, oder in der pharmazeutischen oder kosmetischen Industrie.

21. Co-Produkt, dargestellt durch die im Verfahren nach einem der Ansprüche 1 bis 9 abgetrennte feste Phase, dadurch gekennzeichnet, daß es frei von β-Casein ist.

22. Anwendung des durch die im Verfahren nach einem der Ansprüche 1 bis 9 abgetrennte feste Phase dargestellten Coprodukts als Nahrungsmittel oder Nahrungsergänzung in der Lebensmittelindustrie oder diätetischen Industrie, insbesondere Molkerei oder Käserei, oder in der pharmazeutischen oder kosmetischen Industrie.

**Patentansprüche für folgenden Vertragsstaat : ES**

1. Verfahren zur Herstellung von $\beta$-Casein, dadurch gekennzeichnet, daß man Lab-Casein verwendet, wie es bei der enzymatischen Koagulation von Milch anfällt, worin das $\kappa$-Kasein zu para-$\kappa$-Casein hydrolysiert wird.

2. Verfahren zur Herstellung von $\beta$-Casein aus einer Suspension oder Lösung von Lab-Casein, wie es bei der enzymatischen Koagulation einer Säugetiermilch anfällt, dadurch gekennzeichnet, daß es die Stufen der Abkühlung der Suspension oder Lösung des Lab-Caseins auf eine Temperatur in der Größenordnung von -2°C bis +10°C, vorzugsweise +2°C bis +5°C,
   - die Einstellung des pH-Wertes auf einen Wert von 4,00 bis 5,00,
   - die Auftrennung der solcherart abgekühlten und angesäuerten Suspension oder Lösung des Lab-Caseins in zwei Phasen, um eine feste Phase und eine flüssige Phase zu erhalten, wobei die letztgenannte das $\beta$-Casein enthält,
   umfaßt.

3. Verfahren nach Anspruch 1 oder 2, dadurch gekennzeichnet, daß das Lab-Casein aus der enzymatischen Koagulation von Säugetiermilch durch ein Enzymgemisch vom Labtyp oder durch ein Enyzm tierischer, pflanzlicher, bakterieller, pilzlicher Herkunft oder durch ein Gemisch dieser verschiedenen Enzyme stammt, die die Hydrolyse des Lab-Caseins ermöglichen.

4. Verfahren nach einem der Ansprüche 1 bis 3, dadurch gekennzeichnet, daß die Konzentration des Lab-Caseins, das in der Suspension oder Lösung des Lab-Caseins vorliegt, in der Größenordnung von 1 bis 10%, vorzugsweise von 4 bis 7 % liegt.

5. Verfahren nach einem der vorstehenden Ansprüche, dadurch gekennzeichnet, daß das Lab-Casein mit Wasser oder mit einer Salzlösung, gebildet aus Wasser und 0,1 bis 4 % Salzen, die unter Natriumchlorid, Kaliumchlorid oder Ammoniumchlorid, Natriumzitrat, Kaliumzitrat oder Ammoniumzitrat, Natriumoxalat, Kaliumoxalat oder Ammoniumoxalat, Natriumphosphat, Kaliumphosphat oder Ammoniumphosphat, einzeln oder in verschiedenen Gemischen, ausgewählt sind, verdünnt wird.

6. Verfahren nach einem der vorstehenden Ansprüche, dadurch gekennzeichnet, daß der pH-Wert der abgekühlten Lösung durch Zugabe einer organischen Säure oder einer Mineralsäure oder eines Gemisches dieser beiden Säuretypen eingestellt wird.

7. Verfahren nach Anspruch 6, dadurch gekennzeichnet, daß man als organische Säure Essigsäure, Zitronensäure, Milchsäure, Oxalsäure, einzeln oder im Gemisch, und als Mineralsäure Chlorwasserstoffsäure, Schwefelsäure, Phosphorsäure, Salpetersäure, einzeln oder im Gemisch, verwendet.

8. Verfahren nch einem der Ansprüche 2 bis 7, dadurch gekennzeichnet, daß die Ansäuerungsstufe der Lösung oder Suspension des Lab-Caseins vor der Temperaturregelungsstufe dieser Lösung oder Suspension auf Werte von -2°C bis +10°C ausgeführt werden kann.

9. Verfahren nach einem der Ansprüche 7 bis 8, dadurch gekennzeichnet, daß die abgekühlte und angesäuerte Suspension oder Lösung des Lab-Caseins in zwei Phasen aufgetrennt wird: eine flüssige Phase, die das $\beta$-casein enthält, und eine sedimentierbare feste Phase, die die anderen Caseine enthält, wobei diese Auftrennung insbesondere durch Dekantieren bei Atmosphärendruck oder durch Klärdekantation oder auch durch Zentrifugieren, gegebenenfalls unter gleichzeitiger Kühlung, ausgeführt wird.

10. Vorrichtung zur Durchführung des Verfahrens, wie in einem der Ansprüche 1 bis 9 definiert, dadurch gekennzeichnet, daß sie umfaßt:
    - einen Reaktor (10), in welchen die Lösung oder Suspension des aus der enzymatischen Koagulation von Säugetiermilch stammenden Lab-Caseins (C) eingeführt wird;
    - Kühlmittel, die eine Aufrechterhaltung der Temperatur der Suspension im Reaktor auf einem Wert von -2°C bis +10°C, vorzugsweise +2°C bis +5°C, gestatten;

- einen pH-Stat (16), der unter der Kontrolle eines pH-Meters (22) die Regelung der Säurenmenge gestat-tet, die der im Reaktor enthaltenen Lösung oder Suspension des Lab-Caseins zugesetzt wird; und

- Mittel, die eine Trennung der wäßrigen Phase und der sedimentierbaren Phase gestatten, die in dem vom Reaktor kommenden Gemisch enthalten sind.

11. Vorrichtung nach Anspruch 10, dadurch gekennzeichnet, daß der Reaktor vom Doppelmanteltyp ist, wobei im Außenmantel eine Kühllösung umläuft und die Suspension oder Lösung des Lab-Caseins mit Hilfe eines Rührsystems (12) mit variierbarer Geschwindigkeit einer schwachen Bewegung unterworfen wird.

12. Vorrichtung nach einem der Ansprüche 10 oder 11, dadurch gekennzeichnet, daß der pH-Wert der im Reaktor (10) enthaltenen Lösung oder Suspension des Lab-Caseins (C) mit dem pH-Stat (16) konstant gehalten wird, dem ein pH-Sollwert eingegeben wird, wobei eine Dosierpumpe (20) die Einstellung des pH-Werts der Lösung oder Suspension in Abhängigkeit vom Sollwert durch Abgabe der erforderlichen Säuremenge ermöglicht.

13. Vorrichtung nach einem der Ansprüche 10 bis 12, dadurch gekennzeichnet, daß die das β-casein enthaltende flüssige Phase durch Absaugen, gegebenenfalls mit Hilfe einer Zentrifugalpumpe oder Verdrängerpumpe, nach einem Sedimentieren der festen Phase durch Dekantieren in einem Behälter (30) bei atmosphärischem Druck gewonnen wird.

14. Vorrichtung nach Anspruch 13, dadurch gekennzeichnet, daß das Dekantieren bei atmosphärischem Druck in einem Behälter (30) erfolgt, der eine im Bereich der Grenzschicht zwi-schen der flüssigen Phase und der sedimentierten Phase an-geordnete Öffnung trägt, um die Gewinnung der oberen, flüssigen, das β-Casein enthaltenden Phase sicherzustellen.

15. Vorrichtung nach einem der Ansprüche 10 bis 12, dadurch gekennzeichnet, daß die Trennung der beiden Phasen mit Hilfe einer kontinuierlich oder diskontinuierlich betriebenen Zentrifuge (38), mit Hilfe einer kontinuierlich oder diskontinuierlich arbeitenden Klärvorrichtung, die selbstentschlammend ist oder auch nicht, oder mit Hilfe einer kontinuierlich oder diskontinuierlich betriebenen Dekantiervorrichtung (44) vorgenommen wird.

16. Vorrichtung nach Anspruch 15, dadurch gekennzeichnet, daß die Beschleunigung der Mittel zur Auftrennung der beiden Phasen von 500 bis 8.000 g beträgt.

17. Vorrichtung nch einem der Ansprüche 10 bis 16, dadurch gekennzeichnet, daß die vom Reaktor (10) kommende, angesäuerte und abgekühlte Lösung den Trennvorrichtungen durch Schwerkraft, durch eine zwischengeschaltete gekühlte oder ungekühlte Leitung oder mit Hilfe einer Verdrängerpumpe (26) oder Zentrifugalpumpe (28) den Trennmitteln zugeführt wird.

18. Vorrichtung nch einem der Ansprüche 10 bis 17, dadurch gekennzeichnet, daß sie kontinuierlich oder diskontinuierlich betrieben wird.

19. Verfahren nach einem der Ansprüche 1 bis 9, dadurch gekennzeichnet, daß man ein β-casein erhält, das einen Reinheitsgrad von größer als ungefähr 90%, bezogen auf das Gesamtproteinmaterial, aufweist, das frei von chemischen Additiven wie Denaturierungsmitteln, Ausfällungsmitteln oder Harnstoff ist, das darüber hinaus praktisch zur Gänze frei von κ-Casein ist und einen pH-Wert nahe dem Neutralpunkt aufweist.

20. Anwendung des β-Caseins nach Anspruch 19 als Nahrungsmittel oder Nahrungsmittelergänzung in der Lebensmittelindustrie und diätetischen Industrie, insbesondere Molkerei oder Käserei, oder in der pharmazeutischen oder kosmetischen Industrie.

21. Verfahren nach einem der Ansprüche 1 bis 9, dadurch gekennzeichnet, daß man eine feste Phase oder ein Co-Produkt erhält, die bzw. das frei von β-Casein ist.

22. Anwendung des durch die im Verfahren nach einem der Ansprüche 1 bis 9 abgetrennte feste Phase dargestellten Coprodukts als Nahrungsmittel oder Nahrungsergänzung in der Lebensmittelindustrie oder diätetischen Industrie, insbesondere Molkerei oder Käserei, oder in der pharmazeutischen oder kosmetischen Industrie.

## Claims

**Claims for the following Contracting States : AT, BE, CH, DE, DK, FR, GB, GR, IT, LI, LU, NL, SE**

1. A method for obtaining beta casein, characterized in that use is made of rennet casein, such as produced by the enzymatic curdling of milk, wherein the kappa casein is hydrolyzed into para kappa casein.

2. A method for obtaining beta casein from a suspension or solution of rennet casein, for example as produced by the enzymatic curdling of a mammal milk, characterized in that it comprises the steps of cooling the suspension or solution of rennet casein at a temperature of the order of -2°C and +10°C, preferably of +2°C to +5°C,
   - adjusting the pH to a value of 4.00 to 5.00,
   - separating the suspension or solution of rennet casein thus cooled and acidified into two phases, so as to obtain a solid phase and a liquid phase, the latter containing the beta casein.

3. A method according to Claim 1 or 2, characterized in that the rennet casein is produced by the enzymatic curdling of the mammal milk by a mixture of enzymes of rennet type or by an enzyme of animal, vegetal, bacterial or fungal origin, or a mixture of said different enzymes enabling the rennet casein to be hydrolyzed.

4. A method according to one of Claims 1 to 3, characterized in that the concentration of the rennet casein present in the suspension or solution of rennet casein is of the order of 1 to 10 %, preferably of 4 to 7 %.

5. A method according to any of the preceding Claims, characterized in that the rennet casein is diluted in water or in a saline solution formed by water and 0.1 to 4 % of salts selected from sodium, potassium or ammonium chlorides, sodium, potassium or ammonium citrates, sodium, potassium or ammonium oxalates, sodium, potassium or ammonium phosphates, separately or in various mixtures.

6. A method according to any of the preceding Claims, characterized in that the pH of the cooled solution is adjusted by the addition of an organic acid or a mineral acid or a mixture of said two types of acids.

7. A method according to Claims 6, characterized in that the organic acid used is acetic acid, citric acid, lactic acid, oxalic acid, separately or in a mixture, the mineral acid used being hydrochloric acid, sulphuric acid, phosphoric acid, nitric acid, separately or in a mixture.

8. A method according to one of Claims 2 to 7, characterized in that the step of acidification of the solution or suspension of rennet casein can be performed prior to the stage of controlling the temperature of said solution or suspension to values from -2°C to +10°C.

9. A method according to one of Claims 7 or 8, characterized in that the cooled and acidified suspension or solution of rennet casein is separated into two phases: a liquid phase containing the beta casein and a sedimentable solid phase containing the other caseins, said separation being performed more particularly by decantation at atmospheric pressure of by clarification decantation or else by centrifugation, possibly with simultaneous cooling.

10. A device for the performance of the method as defined in any of Claims 1 to 9, characterized in that it comprises :
    - a reactor (10) into which the solution or suspension of rennet casein (C) produced by the enzymatic curdling of mammal milk is introduced ;
    - cooling means enabling the temperature of the suspension in the reactor to be maintained at a value of -2°C to +10°C, preferably of +2°C to +5°C ;
    - a pH-stat enabling the quantity of acids added to the solution or suspension of rennet casein contained in the reactor to be regulated, under the control of the pH-meter (22), and
    - means for ensuring the separation of the liquid and sedimentable phases contained in the mixture originating from said reactor.

11. A device according to Claim 10, characterized in that the reactor is of the jacketed type, a refrigerating solution circulating in the jacket and the suspension or solution of rennet casein being subjected to a slight agitation by means of a variable speed agitator system (12).

12. A device according to one of Claims 10 or 11, characterized in that the pH of the solution or suspension of rennet casein (C) contained in the reactor (10) is kept constant by said pH-stat (16), which is given a required pH value, a metering pump (20) enabling the pH of the solution or suspension to be adjusted in accordance with the required value by supplying the necessary quantity of acid.

13. A device according to any of Claims 10 to 12, characterized in that the liquid phase containing the beta casein is collected by siphoning (36), possibly by means of a centrifugal or volumetric pump, after sedimentation of the solid phase by decantation into an enclosure (30) at atmospheric pressure.

14. A device according to Claim 13, characterized in that decantation at atmospheric pressure is performed in a receptacle (30), comprising an orifice situated in the zone of the interface between the liquid phase and the sedimented phase, to ensure the recovery of the upper, liquid phase containing the beta casein.

15. A device according to any of Claims 10 to 12, characterized in that the separation of the two phases is performed by means of continuously or intermittently operating centrifuge (38), or a continuously or intermittently operating self-clearing or non-self-clearing clarifier, or a continuously or intermittently operating decanter (44).

16. A device according to Claim 15, characterized in that the acceleration of the means for separating the two phases is from 500 to 8000 g.

17. A device according to any of Claims 10 to 16, characterized in that the acidified and cooled solution coming from the reactor (10) is transmitted to the separating means by gravity, via a cooled or uncooled conduit, or by means of a volumetric pump (26) or a centrifugal pump (28).

18. A device according to any of Claims 10 to 17, characterized in that it operates continuously or intermittently.

19. Beta casein as obtained by the method according to any of Claims 1 to 9, characterized in that it has a degree of purity higher than approximately 90 % in relation to the total protein material, it is free from chemical additives such as denaturing agents, precipitating agents or urea, it is moreover substantially totally free from kappa casein, and it has a pH close to neutrality.

20. Application of the beta casein according to Claim 19, as a food or food complement in the agricultural-foodstuffs or dietetic industry, more particularly the milk or cheese-producing industry, or in the pharmaceutical or cosmetics industry.

21. A coproduct, for example as represented by the solid phase separated in the process according to any of Claims 1 to 9, characterized in that it is free from beta casein.

22. Application of the coproduct represented by solid phase separated in the process according to any of Claims 1 to 9, as a food or food completment in the agricultural-foodstuffs or dietetic industry, more particularly the milk or cheese-producing industry, or in the pharmaceutical or cosmetics industry.

**Claims for the following Contracting State : ES**

1. A method for obtaining beta casein, characterized in that use is made of rennet casein, such as produced by the enzymatic curdling of milk, wherein the kappa casein is hydrolyzed to form para kappa casein.

2. A method for obtaining beta casein from a suspension or solution of rennet casein, such as produced by the enzymatic curdling of a mammal milk, characterized in that it comprises the steps of cooling the suspension or solution of rennet casein at a temperature of the order of -2°C and +10°C, preferably between +2°C and +5°C,
   - adjusting the pH to a value of 4.00 to 5.00,
   - separating the suspension or solution of rennet casein thus cooled and acidified into two phases, so as to obtain a solid phase and a liquid phase, the latter containing the beta casein.

3. A method according to Claim 1 or 2, characterized in that the rennet casein is produced by the enzymatic curdling of the mammal milk by a mixture of enzymes of rennet type or by an enzyme of animal, vegetal, bacterial or fungal origin, or a mixture of said different enzymes enabling the rennet casein to be hydrolyzed.

4. A method according to one of Claims 1 to 3, characterized in that the concentration of the rennet casein present in the suspension or solution of rennet casein is of the order of 1 to 10 %, preferably of 4 to 7 %.

5. A method according to any of the preceding Claims, characterized in that the rennet casein is diluted in water or in a saline solution formed by water and 0.1 to 4 % of salts selected from sodium, potassium or ammonium chlorides, sodium, potassium or ammonium citrates, sodium, potassium or ammonium oxalates, sodium, potassium or ammonium phosphates, separately or in various mixtures.

6. A method according to any of the preceding Claims, characterized in that the pH of the cooled solution is adjusted by the addition of an organic acid or a mineral acid or a mixture of said two types of acids.

7. A method according to Claim 6, characterized in that the organic acid used is acetic acid, citric acid, lactic acid, oxalic acid, separately or in a mixture, the mineral acid used being hydrochloric acid, sulphuric acid, phosphoric acid, nitric acid, separately or in a mixture.

8. A method according to one of Claims 2 to 7, characterized in that the step of acidification of the solution or suspension of rennet casein can be performed prior to the stage of controlling the temperature of said solution or suspension to values from -2°C to +10°C.

9. A method according to one of Claims 7 or 8, characterized in that the cooled and acidified suspension or solution of rennet casein is separated into two phases: a liquid phase containing the beta casein and a sedimentable solid phase containing the other caseins, said separation being performed more particularly by decantation at atmospheric pressure of by clarification decantation or else by centrifugation, possibly with simultaneous cooling.

10. A device for the performance of the method as defined in any of Claims 1 to 9, characterized in that it comprises :
    - a reactor (10) into which the solution or suspension of rennet casein (C) produced by the enzymatic curdling of mammal milk is introduced ;
    - cooling means enabling the temperature of the suspension in the reactor to be maintained at a value of -2°C to +10°C, preferably between +2°C to +5°C ;
    - a pH-stat (16) enabling the quantity of acids added to the solution or suspension of rennet casein contained in the reactor to be regulated, under the control of the pH-meter (22), and
    - means for ensuring the separation of the liquid and sedimentable phases contained in the mixture originating from said reactor.

11. A device according to Claim 10, characterized in that the reactor is of the jacketed type, a refrigerating solution circulating in the external jacket and the suspension or solution of rennet casein being subjected to a slight agitation by means of a variable speed agitator system (12).

12. A device according to one of Claims 10 or 11, characterized in that the pH of the solution or suspension of rennet casein (C) contained in the reactor (10) is kept constant by said pH-stat (16), which is given a required pH value, a metering pump (20) enabling the pH of the solution or suspension to be adjusted in accordance with the required value by supplying the necessary quantity of acid.

13. A device according to any of Claims 10 to 12, characterized in that the liquid phase containing the beta casein is collected by siphoning (36), possibly by means of a centrifugal or volumetric pump, after sedimentation of the solid phase by decantation into an enclosure (30) at atmospheric pressure.

14. A device according to Claim 13, characterized in that decantation at atmospheric pressure is performed in a receptacle (30), comprising an orifice situated in the zone of the interface between the liquid phase and the sedimented phase, to ensure the recovery of the upper, liquid phase containing the beta casein.

15. A device according to any of Claims 10 to 12, characterized in that the separation of the two phases is performed by means of continuously or intermittently operating centrifuge (38), or a continuously or intermittently operating self-clearing or non-self-clearing clarifier, or a continuously or intermittently operating decanter (44).

16. A device according to Claim 15, characterized in that the acceleration of the means for separating the two phases is from 500 to 8000 g.

17. A device according to any of Claims 10 to 16, characterized in that the acidified and cooled solution coming from the reactor (10) is transmitted to the separating means by gravity, via a cooled or uncooled conduit, or by means of a volumetric pump (26) or a centrifugal pump (28).

18. A device according to any of Claims 10 to 17, characterized in that it operates continuously or intermittently.

19. Beta casein as obtained by the method according to any of Claims 1 to 9, characterized in that it has a degree of purity higher than approximately 90 % in relation to the total protein material, it is free from chemical additives such as denaturing agents, precipitating agents or urea, it is moreover substantially totally free from kappa casein, and it has a pH close to neutrality.

20. Application of the beta casein according to Claim 19, as a food or food complement in the agricultural-foodstuffs or dietetic industry, more particularly the milk or cheese-producing industry, or in the pharmaceutical or cosmetics industry.

21. The process according to any of Claims 1 to 9, characterized in that a solid phase or co-product, free from beta casein, is obtained.

22. Application of the coproduct represented by solid phase separated in the process according to any of Claims 1 to 9, as a food or food completment in the agricultural-foodstuffs or dietetic industry, more particularly the milk or cheese-producing industry, or in the pharmaceutical or cosmetics industry.

Figure 1

Caséine
Présure                                    Sels

                    △

                              Eau

Solution de Caséine Présure
1 à 10 %

Acide                          Refroidissement - 2° C/10° C

pH-stat

Décantation              Centrifugeuse              Décanteur
naturelle                Clarificateur

Phase solide                          Phase liquide
Sédiments

Caséines non solubilisées              Caséine β

FIG.2

FIG.3

FIG. 4

FIG.5

19

Figure 6